# EUROPEAN PATENT APPLICATION

(11) **EP 4 501 353 A1**
(43) Date of publication of application: **05.02.2025**
(21) Application number: 23780411.7
(22) Date of filing: 27.03.2023
(51) Int. Cl.: A61K 39/395, C07D 257/02, C07K 1/13, C07K 16/00, A61K 47/68, C12N 15/13, A61K 51/10, A61K 31/555

(54) **METHOD FOR PRODUCING COMPLEX**

(30) Priority: 30.03.2022 JP 2022057723
(71) Applicant: Nihon Medi-Physics Co., Ltd., Tokyo 136-0075 (JP)
(72) Inventor: KISHIMOTO, Satoshi, Tokyo 136-0075 (JP); OTSUKA, Yuta, Tokyo 136-0075 (JP); IMAI, Tomoyuki, Tokyo 136-0075 (JP); NARITA, Yudai, Tokyo 136-0075 (JP); TAKEDA, Takuya, Tokyo 136-0075 (JP)
(74) Representative: TBK
(86) International application number: PCT/JP2023/012320
(87) International publication number: WO 2023/190402

(57) **Abstract**

A method for producing a conjugate of a radioactive metal nuclide and an antibody is provided, and the method includes a step of reacting a radioactive metal nuclide with an antibody conjugated with a ligand compound in a reaction solution containing water to coordinate the radioactive metal nuclide to the ligand compound, and the reaction solution does not contain a pH buffer and has a pH of 2 or more and 10 or less. The radioactive metal nuclide is preferably at least one selected from ⁶⁸Ga, ⁸⁹Zr, ⁹⁰Y, ¹¹¹In, ¹⁷⁷Lu, and ²²⁵Ac. It is also preferable that the radioactive metal nuclide be ⁸⁹Zr and the reaction solution have a pH of 2 or more and 4 or less.

## Description

### Technical Field

The present invention relates to a method for producing a conjugate.

### Background Art

For the purpose of use in reagents and diagnostics for detection of target molecules, or use in pharmaceuticals for treatment of diseases, studies have been conducted on the labeling rate of a conjugate in which a radioactive metal is coordinated to a ligand compound that is conjugated with an antibody.

Patent Literature 1 discloses that radioactive zirconium and DTPA, which is a kind of ligand compound, are reacted in acidic physiological saline to form a Zr complex.

Patent Literature 2 discloses that radioactive zirconium and DOTA, which is a kind of ligand compound, are heated and reacted in a neutral HEPES buffer solution to form a Zr complex.

Patent Literature 3 discloses that radioactive actinium and rituximab conjugated with DOTA, which is a kind of ligand compound, are reacted in a basic Tris buffer solution to form an Ac complex.

Patent Literature 4 discloses that radioactive actinium and HuM-195, which is a recombinant IgG1 antibody conjugated with DOTA as a kind of ligand compound, are reacted in an acetate buffer solution containing gentisic acid at pH 5.5 to 7.0 to form an Ac complex.

Non Patent Literature 1 describes a method in which radioactive zirconium and DOTA as a ligand compound are reacted in a buffer solution to form a Zr complex.

### Citation List

### Patent Literature

Patent Literature 1: US 2014/147381 A
Patent Literature 2: US 2019/038785 A
Patent Literature 3: US 2015/0157742 A
Patent Literature 4: US 2012/0220754 A

### Non Patent Literature

Non Patent Literature 1: Pandya et al, Chem Sci. 2017; 8 (3): 2309-14

### Summary of Invention

However, under the conditions disclosed in Patent Literature 1 and 2 and Non Patent Literature 1, complexation does not proceed well between a ligand compound and a radioisotope, and a sufficient labeling rate cannot be achieved by the radioisotope for an antibody conjugated with the ligand compound (hereinafter, referred to as a ligand-conjugated antibody). Therefore, reaction conditions have been desired under which a high labeling rate can be stably achieved by a radioisotope for a ligand-conjugated antibody. Therefore, an object of the present invention is to provide a method for producing a conjugate that includes a radioisotope, a ligand compound, and an antibody and is capable of imparting a high labeling rate by the radioisotope to a ligand-conjugated antibody.

As a result of intensive studies to solve the above problem, the present inventors have found that if a radioactive metal nuclide and an antibody conjugated with a ligand compound are reacted in a reaction solution not containing a pH buffer but containing water and having a pH of 2 or more and 10 or less, a high labeling rate can be imparted by the radioisotope to the ligand-conjugated antibody.

That is, the present invention provides a method for producing a conjugate of a radioactive metal nuclide and a ligand-conjugated antibody, the method including a step of reacting a radioactive metal nuclide with a ligand-conjugated antibody in a reaction solution containing water to coordinate the radioactive metal nuclide to the ligand compound, wherein the reaction solution does not contain a pH buffer and has a pH of 2 or more and 10 or less.

According to the present invention, it is possible to provide a method for producing a conjugate that includes a radioisotope, a ligand compound, and an antibody and is capable of imparting a high labeling rate by the radioisotope to a ligand-conjugated antibody.

### Brief Description of Drawing

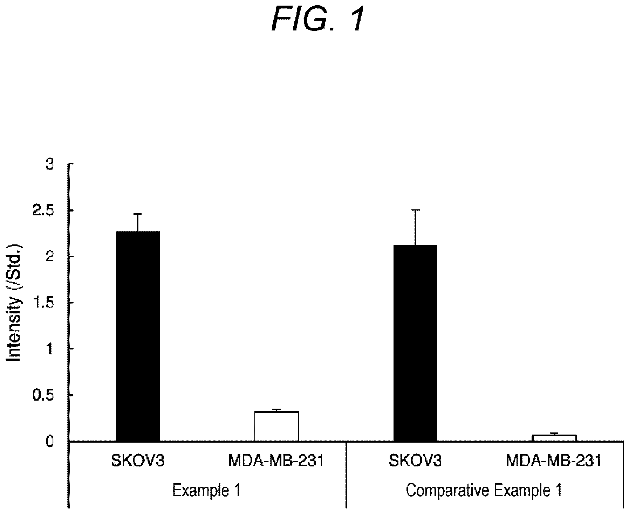

Fig. 1 is a graph showing the antigen-binding activity of conjugates produced in Example 1 and Comparative Example 1 to SK-OV-3 tumor sections and MDA-MB-231 tumor sections.

### Description of Embodiments

Hereinafter, the present invention will be described on the basis of preferred embodiments of the present invention.

The method for producing a conjugate of the present invention includes a step of reacting a radioactive metal nuclide with a ligand-conjugated antibody in a reaction solution containing water to coordinate the radioactive metal nuclide to the ligand compound. The step is performed in a state in which the reaction solution contains water but does not contain a pH buffer, and has a pH of 2 or more and 10 or less. Through the step, the radioisotope is bound to the ligand compound conjugated with the antibody, by a combination of bonds such as a covalent bond and an ionic bond in addition to a coordinate bond, and thus a conjugate is obtained in which the antibody is directly labeled.

From the viewpoint of enhancing the labeling rate, the radioisotope used in the step is preferably used in the form of a compound that can ionize in water, and more preferably used in the form of a radioisotope ion (hereinafter, these forms are also collectively referred to as a "radioisotope source"). As the radioisotope source, for example, a radioisotope ion-containing liquid can be used in which a radioisotope ion is dissolved or dispersed in a solvent mainly containing water.

The nuclide of the radioisotope is preferably at least one selected from ⁶⁸Ga, ⁸⁹Zr, ⁹⁰Y, ¹¹¹In, ¹⁷⁷Lu, and ²²⁵Ac, and particularly preferably at least one of ⁸⁹Zr or ²²⁵Ac from the viewpoint of high labeling rate and easy labeling.

⁸⁹Zr is a β⁺ ray decay nuclide and is an electron capture decay nuclide. ⁸⁹Zr can be produced, for example, by a nuclear reaction of ⁸⁹Y(p, n)⁸⁹Zr using a cyclotron. Specifically, a solution obtained by dissolving a ⁸⁹Y target after proton irradiation with an acid is passed through a column cartridge or the like carrying a collector capable of adsorbing ⁸⁹Zr. Thereafter, the column cartridge is washed with a solvent such as water, then an oxalic acid aqueous solution is passed through the column cartridge, and thus ⁸⁹Zr ions can be eluted and collected as a solution.

²²⁵Ac can be generated by any of milking from a Th-229 generator, Th-232 spallation, Ra-226 neutron addition (a method in which a nuclear reaction of ²²⁶Ra(n, 2n)²²⁵Ra and β-decay of ²²⁵Ra are performed), Ra-226 photonuclear reaction (a method in which a nuclear reaction of ²²⁶Ra(γ, n)²²⁵Ra and β-decay of ²²⁵Ra are performed), or Ra-226 nuclear transformation (nuclear reaction of ²²⁶Ra(p, 2n)²²⁵Ac using a cyclotron). In the case of using Ra-226 nuclear transformation, the generated ²²⁵Ac can be purified by, for example, separation and purification from a ²²⁶Ra target, and purified ²²⁵Ac can be obtained by dissolving a ²²⁶Ra target containing ²²⁵Ac with an acid or the like, adding an alkali to the solution to precipitate a salt containing ²²⁵Ac, and separating and purifying the salt.

The ligand compound used in the step is not particularly limited as long as it is a compound that can be coordinated with a radioisotope, and examples of the ligand compound include the following organic compounds and compounds including a structure derived from a compound described below.

CB-TE2A (1,4,8,11 -tetraazabicyclo[6.6.2]hexadecane-4,11-diacetic acid)
CDTA (cyclohexane-trans-1,2-diamine tetra-acetic acid)
CDTPA (4-cyano-4-[[(dodecylthio)thioxomethyl]thio]-pentanoic acid)
DOTA (1,4,7,10-tetraazacyclododecane-1,4,7,10-tetraacetic acid)
DOTMA ((1R,4R,7R,10R)-α,α',α",α‴-tetramethyl-1,4,7,10-tetraazacyclododecane-1,4,7,10-tetraacetic acid)
DOTAM (1,4,7,10-tetrakis(carbamoylmethyl)-1,4,7,10-tetraazacyclododecane)
DOTA-GA (α-(2-carboxyethyl)-1,4,7,10-tetraazacyclododecane-1,4,7,10-tetraacetic acid)
DOTP (((1,4,7,10-tetraazacyclododecane-1,4,7,10-tetrayl)tetrakis(methylene))tetraphosphonic acid)
DOTMP (1,4,7,10-tetraazacyclododecane-1,4,7,10-tetrakis(methylenephosphonic acid))
DOTA-4AMP (1,4,7,10-tetraazacyclododecane-1,4,7,10-tetrakis(acetamidomethylenephosphonic acid)
DO2P (tetraazacyclododecane dimethanephosphonic acid)

### Deferoxamine (DFO)

DTPA (N,N-bis[2-[bis(carboxymethyl)amino]ethyl]-glycine)
DTPA-BMA (5,8-bis(carboxymethyl)-11-[2-(methylamino)-2-oxoethyl]-3-oxo-2,5,8,11-tetraazatridecan-13-oic acid)
EDTA (2,2',2",2‴-(ethane-1,2-diylbis(azanetriyl))tetraacetic acid)
NOTA (1,4,7-triazacyclononane-1,4,7-triacetic acid)
OTP (1,4,7-triazacyclononane-1,4,7-triyltris(methylenephosphonic acid)
TETPA (1,4,8,11-tetraazacyclotetradecane-1,4,8,11-tetrapropionic acid)
TETA (1,4,8,11-tetraazacyclotetradecane-N,N',N",N‴-tetraacetic acid)
TTHA (3,6,9,12-tetrakis(carboxymethyl)-3,6,9,12-tetraazatetradecanedioic acid)
HEHA (1,2,7,10,13-hexaazacyclooctadecane-1,4,7,10,13,16-hexaacetic acid)
1,2-HOPO (N,N',N",N‴-tetra(1,2-dihydro-1-hydroxy-2-oxopyridine-6-carbonyl)-1,5,10,14-tetraazatetradecane)
PEPA (1,4,7,10,13-pentaazacyclopentadecane-N,N',N",N‴,Nʺʺ-penta-acetic acid)
H4octapa (N,N'-bis(6-carboxy-2-pyridylmethyl)-ethylenediamine-N,N'-diacetic acid)
H2bispa2 (6,6'-({9-hydroxy-1,5-bis(methoxycarbonyl)-2,4-di(pyridine-2-yl)-3,7-diazabicyclo[3.3. 1]nonane-3,7-diyl}bis(-methylene))dipicolinic acid)
H2dedpa (1,2-[{6-(carboxy)-pyridin-2-yl}-methylamino]ethane)
H2macropa (6-(1,4,10,13-tetraoxa-7,16-diazacyclooctadecan-N,N'-methyl)picolinic acid)
H5decapa (N,N" -bis(6-carboxy-2-pyridylmethyl)-diethylenetriamine-N,N',N"-triacetic acid)
H6phospa (N,N' -(methyl enephosphonate)-N,N' - [6-(methoxycarbonyl)pyridin-2-yl]-methyl-1,2-diaminoethane)
HP-D03A (hydroxypropyltetraazacyclododecanetriacetic acid)

Among these, the ligand compound used in the step is preferably an organic compound having a structure represented by Formula (1) described below.

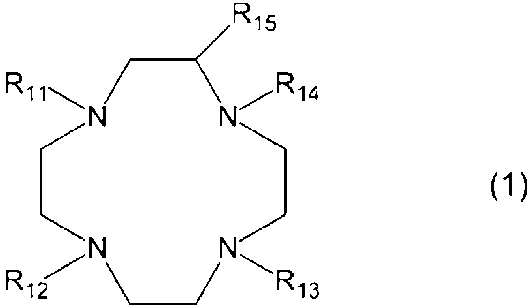

In Formula (1), R₁₁, R₁₃, and R₁₄ each independently represent a group including -(CH₂)ₚCOOH, -(CH₂)ₚC₅H₄N, -(CH₂)ₚPO₃H₂, or -(CH₂)ₚCONH₂. Each p is independently an integer of 0 or more and 3 or less.

In Formula (1), one of R₁₂ or R₁₅ represents a hydrogen atom, a carboxyl group, or a carboxyalkyl group having 2 or 3 carbon atoms, the other represents a substituent that is to be conjugated with the antibody, R₁₅ is a hydrogen atom when R₁₂ is a substituent that is to be conjugated with the antibody, and R₁₅ is a substituent that is to be conjugated with the antibody when R₁₂ is not a substituent that is to be conjugated with the antibody.

The ligand compound used in the step is more preferably 1,4,7,10-tetraazacyclododecane-1,4,7,10-tetraacetic acid (DOTA) or a compound including a structure derived from a derivative of DOTA, and specifically, the ligand compound is still more preferably one compound described below or a compound including a structure derived from a compound described below. The ligand compound used in the step is preferably water-soluble.

DOTA (1,4,7,10-tetraazacyclododecane-1,4,7,10-tetraacetic acid)
DOTMA ((1R,4R,7R,10R)-α,α',α",α‴-tetramethyl-1,4,7,10-tetraazacyclododecane-1,4,7,10-tetraacetic acid)
DOTAM (1,4,7,10-tetrakis(carbamoylmethyl)-1,4,7,10-tetraazacyclododecane)
DOTA-GA (α-(2-carboxyethyl)-1,4,7,10-tetraazacyclododecane-1,4,7,10-tetraacetic acid)
DOTP (((1,4,7,10-tetraazacyclododecane-1,4,7,10-tetrayl)tetrakis(methylene))tetraphosphonicacid)
DOTMP (1,4,7,10-tetraazacyclododecane-1,4,7,10-tetrakis(methylenephosphonic acid))
DOTA-4AMP (1,4,7,10-tetraazacyclododecane-1,4,7,10-tetrakis(acetamidomethylenephosphonic acid)
DO2P (tetraazacyclododecane dimethanephosphonic acid)

The reaction solution in the step contains water but does not contain a pH buffer and preferably does not contain an organic solvent.

As the water, water commonly used in the present technical field can be adopted, and for example, distilled water or ion-exchanged water can be used.

The pH buffer exhibits a pH buffering action in a predetermined pH range when dissolved in water. In the present invention, it has been found that if the reaction solution does not contain a pH buffer and if a radioactive metal nuclide and an antibody conjugated with the ligand compound are reacted in the reaction solution, a high labeling rate can be imparted by the radioisotope to the antibody.

The pH buffer that is not contained is a buffer such as sodium acetate, ammonium acetate, phosphoric acid, phosphate buffered physiological saline, a trishydroxymethylaminomethane buffer solution (Tris buffer solution), a 4-(2-hydroxyethyl)-1-piperazineethanesulfonic acid buffer solution (HEPES buffer solution), or a tetramethylammonium acetate buffer solution.

The phrase "not containing a buffer" means that a buffer is not intentionally contained in the reaction solution but may be unavoidably mixed in the reaction solution.

Examples of the organic solvent that is not contained include water-soluble organic solvents and water-insoluble organic solvents.

Examples of the water-soluble organic solvents include protic polar solvents such as methanol and ethanol, and aprotic polar solvents such as acetonitrile, N,N-dimethylformamide, tetrahydrofuran, dimethyl sulfoxide, and acetone.

Examples of the water-insoluble organic solvents include hexane, toluene, and ethyl acetate.

If such an organic solvent is not contained, a step of separating an organic solvent, such as a solid phase extraction method, is not to be performed, and therefore a high labeling rate can be stably achieved while radiolysis due to concentration is avoided.

The phrase "not containing an organic solvent" means that an organic solvent is not intentionally contained in the reaction solution but may be unavoidably mixed in the reaction solution.

In the present invention, the reaction solution has a pH of 2 or more and 10 or less. Specifically, in the case of using, as the radioisotope, ⁸⁹Zr as a radioactive metal nuclide, the pH is preferably 2 or more and 4 or less, and more preferably 3. In the case of using ²²⁵Ac as a radioactive metal nuclide, the pH is preferably 4 or more and 10 or less, and more preferably 6 or more and 9 or less.

If the pH of the reaction solution is set within the above range, when a radioactive metal nuclide and an antibody conjugated with the ligand compound are reacted in the reaction solution, a high labeling rate can be imparted by the radioisotope to the antibody.

The pH of the reaction solution is adjusted to be within the above pH range before starting the reaction. Specifically, an inorganic acid such as hydrochloric acid or a metal hydroxide such as sodium hydroxide is added to water to adjust the pH of the reaction solution.

When a radioactive metal nuclide and a ligand-conjugated antibody are reacted in the reaction solution, the reaction solution may contain the radioactive metal nuclide in advance and then contain the ligand-conjugated antibody for the reaction, or the reaction solution may contain the radioactive metal nuclide and the ligand-conjugated antibody at the same time. Alternatively, the reaction solution may contain the ligand-conjugated antibody in advance and then contain the radioactive metal nuclide.

Next, the reaction pressure is set to, for example, atmospheric pressure, and heating is performed as necessary. By heating, a further improvement in labeling efficiency can be achieved in a short production time. Specifically, the temperature is preferably 30°C or higher and 80°C or lower. The temperature is more preferably 50°C or higher and 80°C or lower in the case of using, as the radioisotope, ⁸⁹Zr as a radioactive metal nuclide, and more preferably 30°C or higher and 70°C or lower in the case of using ²²⁵Ac as a radioactive metal nuclide. Examples of the method of heating include a method of applying heat from the outside of the reaction system using, for example, a block heater, an air heater, a water bath, an oil bath, a microwave, or a mantle heater. A block heater or an air heater is particularly preferably used for heating from the viewpoint of being able to be incorporated into an automatic synthesizer.

The reaction time is preferably 10 minutes or more and 150 minutes or less, and more preferably 15 minutes or more and 120 minutes or less on condition that the reaction temperature is as described above.

The amount of the reaction solution in the step may be appropriately changed according to the production scale, and is practically 0.01 mL or more and 100 mL or less at the start of the step from the viewpoint of practicality in the production step.

From the viewpoint of enhancing the labeling rate with the target radioisotope, the concentration of the antibody in the reaction solution is preferably 1 µmol/ L or more and 2000 µmol/ L or less, more preferably 5 µmol/L or more and 100 µmol/L or less, and still more preferably 10 µmol/L or more and 50 µmol/L or less at the start of the step.

In the present invention, as necessary, to the reaction solution, at least one can be added that is selected from the group of saccharides consisting of sucrose, trehalose, mannitol, maltose, lactose, arabinose, xylose, sorbitol, fructose, glucose, mannose, melezitose, and nystose, and is preferably selected from the group of saccharides consisting of glucose, xylose, sucrose, trehalose, mannitol, and sorbitol. Thus, it is possible to suppress aggregation of the antibody and, consequently, the conjugate including the radioisotope, the ligand compound, and the antibody.

The amount of these aggregation inhibitors added is, for example, preferably 10 mmol/L or more and 2000 mmol/L or less, and more preferably 100 mmol/L or more and 1000 mmol/L or less.

In the finally obtained conjugate, the ligand compound may be connected to the antibody via a linker.

Examples of the antibody include chimeric antibodies, humanized antibodies, and human antibodies, and the antibody is preferably a human IgG, and more preferably human IgG1, IgG2, or IgG4.

Examples of the linker include substituted or unsubstituted alkyl groups, substituted or unsubstituted heteroalkyl groups, polyethylene glycol (PEG) groups, peptides, sugar chains, a disulfide group, and combinations thereof.

The ligand compound preferably is conjugated with the antibody site-specifically, and more preferably at the Fc region, via the linker. In this case, as the linker, a linker can be used that includes a peptide having Formula (2) described below and including 13 or more and 17 or less amino acid residues (hereinafter, also referred to as "antibody-conjugated peptide") and is formed by a crosslinking reaction between the antibody-conjugated peptide modified with a crosslinking agent and the antibody.

For Formula (2), the description will be given assuming that the left side of the amino acid sequence on the paper surface indicates the N-terminal side, and the right side of the amino acid sequence on the paper surface indicates the C-terminal side. In a case where the ligand compound is connected to the antibody via the antibody-conjugated peptide as a linker, the position at which the ligand compound and the antibody-conjugated peptide are linked is not particularly limited, and for example, the ligand compound can be linked directly or indirectly to the N-terminal or the C-terminal, preferably the N-terminal of the antibody-conjugated peptide. The C-terminal of the antibody-conjugated peptide may be modified by amidation or the like in order to, for example, improve the stability.

(Xₐ)-Xₐₐ₁-(X_{b})-Xₐₐ₂-(X_{c})-Xₐₐ₃-(X_{d}) ·· (2)

In Formula (2), Xₐ, X_{b}, X_{c}, and X_{d} represent consecutive Xs of which number is a, consecutive Xs of which number is b, consecutive Xs of which number is c, and consecutive Xs of which number is d, respectively, X represents an amino acid residue having a side chain having neither a thiol group nor a haloacetyl group, a, b, c, and d are each independently an integer of 1 or more and 5 or less and satisfy a + b + c + d ≤ 14, Xₐₐ₁ and Xₐₐ₃ each independently represent an amino acid residue derived from an amino acid having a side chain having a thiol group, or one of Xₐₐ₁ or Xₐₐ₃ represents an amino acid residue derived from an amino acid having a side chain having a thiol group and the other represents an amino acid residue derived from an amino acid having a side chain having a haloacetyl group, Xₐₐ₁ and Xₐₐ₃ are linked to each other, and Xₐₐ₂ is a lysine residue, an arginine residue, a cysteine residue, an aspartic acid residue, a glutamic acid residue, a 2-aminosuberic acid residue, or a diaminopropionic acid residue, and is modified with the crosslinking agent.

Examples of the amino acid residue that can be included in X in Formula (2) include amino acid residues derived from an amino acid such as glycine, alanine, phenylalanine, proline, asparagine, aspartic acid, glutamic acid, arginine, histidine, serine, threonine, tyrosine, or methionine, and X may be an amino acid residue including the same type of amino acids or may be an amino acid residue including different types of amino acids.

In Formula (2), a, b, c, and d are not particularly limited as long as they are a number within the above-described range, but from the viewpoint of the binding stability between the peptide and the antibody, a is preferably an integer of 1 or more and 3 or less, b is preferably an integer of 1 or more and 3 or less, c is preferably an integer of 3 or more and 5 or less, and d is preferably an integer of 1 or more and 3 or less under the condition of a + b + c + d ≤ 14.

Xₐₐ₁ and Xₐₐ₃ are amino acid residues derived from an amino acid having a side chain having a thiol group, and the amino acids may be the same or different. Examples of the amino acid having a side chain having a thiol group include cysteine and homocysteine. Such amino acid residues are preferably bound by a disulfide bond, or the sulfide groups are preferably bound via a linker having Formula (3) described below. In Formula (3), the broken line part represents a binding moiety with a sulfide group.

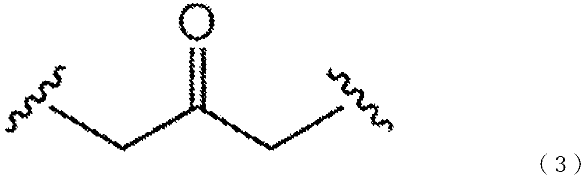

Instead of the above-described combination, combination of Xₐₐ₁ and Xₐₐ₃ may be such that one of Xₐₐ₁ or Xₐₐ₃ is an amino acid residue derived from an amino acid having a side chain having a thiol group and the other is an amino acid residue derived from an amino acid having a side chain having a haloacetyl group. These amino acid residues are bound to each other via a thioether bond. The haloacetyl group has a terminal substituted with a halogen such as iodine, and reacts with the thiol group in the side chain of the other amino acid residue to eliminate the halogen, and thus a thioether bond is formed.

Specific examples of the amino acid sequence of the antibody-conjugated peptide having Formula (2) include peptides described in WO 2016/186206 A, WO 2017/217347 A, and WO 2018/230257 A, and these can also be used.

Among these, the amino acid sequence of the antibody-conjugated peptide is preferably any one of the following sequences (1) to (14), and more preferably the following sequence (1), (2), (13), or (14). In the following amino acid sequences (1) to (14), (Xₐₐ₂) represents a lysine residue, a cysteine residue, an aspartic acid residue, a glutamic acid residue, 2-aminosuberic acid, or diaminopropionic acid, and both (Xₐₐ₁) and (Xₐₐ₃) represent a homocysteine residue. In the following amino acid sequences (1) to (14), amino acids other than (Xₐₐ₁), (Xₐₐ₂), and (Xₐₐ₃) are represented by one-letter codes.

(1) DCAYH(Xₐₐ₂)GELVWCT (SEQ ID NO: 9)
(2) GPDCAYH(Xₐₐ₂)GELVWCTFH (SEQ ID NO: 10)
(3) RCAYH(Xₐₐ₂)GELVWCS (SEQ ID NO: 11)
(4) GPRCAYH(Xₐₐ₂)GELVWCSFH (SEQ ID NO: 12)
(5) SPDCAYH(Xₐₐ₂)GELVWCTFH (SEQ ID NO: 13)
(6) GDDCAYH(Xₐₐ₂)GELVWCTFH (SEQ ID NO: 14)
(7) GPSCAYH(Xₐₐ₂)GELVWCTFH (SEQ ID NO: 15)
(8) GPDCAYH(Xₐₐ₂)GELVWCSFH (SEQ ID NO: 16)
(9) GPDCAYH(Xₐₐ₂)GELVWCTHH (SEQ ID NO: 17)
(10) GPDCAYH(Xₐₐ₂)GELVWCTFY (SEQ ID NO: 18)
(11) SPDCAYH(Xₐₐ₂)GELVWCTFY (SEQ ID NO: 19)
(12) SDDCAYH(Xₐₐ₂)GELVWCTFY (SEQ ID NO: 20)
(13) RGNCAYH(Xₐₐ₂)GQLVWCTYH (SEQ ID NO: 21)
(14) G(Xₐₐ₁)DCAYH(Xₐₐ₂)GELVWCT(Xₐₐ₃)H (SEQ ID NO: 22)

The above embodiments of the present invention include the following technical ideas.
[1] A method for producing a conjugate of a radioactive metal nuclide and an antibody, the method including:
   a step of reacting a radioactive metal nuclide with an antibody conjugated with a ligand compound in a reaction solution containing water to coordinate the radioactive metal nuclide to the ligand compound, wherein
   the reaction solution does not contain a pH buffer and has a pH of 2 or more and 10 or less.
[2] The method according to [1], wherein the radioactive metal nuclide is at least one selected from ⁶⁸Ga, ⁸⁹Zr, ⁹⁰Y, ¹¹¹In, ¹⁷⁷Lu, and ²²⁵Ac.
[3] The method according to [1] or [2], wherein the radioactive metal nuclide is ⁸⁹Zr, and the reaction solution has a pH of 2 or more and 4 or less.
[4] The method according to any one of [1] to [3], wherein the radioactive metal nuclide is ²²⁵Ac, and the reaction solution has a pH of 5 or more and 10 or less.
[5] The method according to any one of [1] to [4], wherein a concentration of the antibody in the reaction solution is 1 µmol/ L or more and 2000 µmol/L or less.
[6] The method according to any one of [1] to [5], wherein the reaction solution is heated to 30°C or higher and 80°C or lower to coordinate the radioactive metal nuclide to the ligand compound.
[7] The method according to any one of [1] to [6], wherein the reaction solution contains at least one aggregation inhibitor selected from the group of saccharides, the group consisting of sucrose, trehalose, mannitol, maltose, lactose, arabinose, xylose, sorbitol, fructose, glucose, mannose, melezitose, and nystose.
[8] The method according to any one of [1] to [7], wherein the ligand compound has Formula (1) described below: 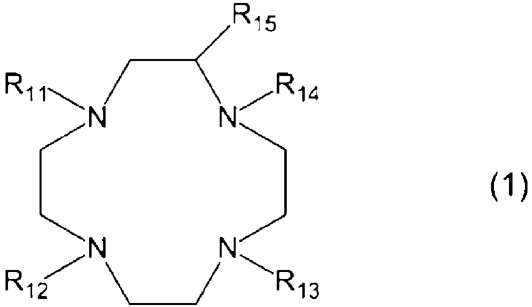 wherein R₁₁, R₁₃, and R₁₄ each independently represent a group including - (CH₂)ₚCOOH, -(CH₂)ₚC₅H₄N, -(CH₂)ₚPO₃H₂, -(CH₂)ₚCONH₂, or-(CHCOOH)(CH₂)ₚCOOH, one of R₁₂ or R₁₅ represents a hydrogen atom, a carboxyl group, or a carboxyalkyl group having 2 or 3 carbon atoms, another of R₁₂ or R₁₅ represents a substituent that is to be conjugated with the antibody, p is an integer of 0 or more and 3 or less, R₁₅ is a hydrogen atom when R₁₂ is a substituent that is to be conjugated with the antibody, and R₁₅ is a substituent that is to be conjugated with the antibody when R₁₂ is not a substituent that is to be conjugated with the antibody.
[9] The method according to any one of [1] to [8], wherein an Fc region of the antibody is site-specifically conjugated with the ligand compound via a linker.
[10] The method according to any one of [1] to [9], wherein
   the antibody is a human IgG antibody, and the linker includes a peptide including 13 or more and 17 or less amino acid residues and is formed by a crosslinking reaction between the peptide modified with a crosslinking agent and the antibody,
   the peptide having Formula (Y) below:

      (Xₐ)-Xₐₐ₁-(X_{b})-Xₐₐ₂-(X_{c})-Xₐₐ₃-(X_{d}) ·· (Y)
   wherein Xₐ, X_{b}, X_{c}, and X_{d} represent consecutive Xs of which number is a, consecutive Xs of which number is b, consecutive Xs of which number is c, and consecutive Xs of which number is d, respectively, X represents an amino acid residue having a side chain having neither a thiol group nor a haloacetyl group, a, b, c, and d are each independently an integer of 1 or more and 5 or less and satisfy a + b + c + d ≤ 14, Xₐₐ₁ and Xₐₐ₃ each independently represent an amino acid residue derived from an amino acid having a side chain having a thiol group, or one of Xₐₐ₁ or Xₐₐ₃ represents an amino acid residue derived from an amino acid having a side chain having a thiol group and another of Xₐₐ₁ or Xₐₐ₃ represents an amino acid residue derived from an amino acid having a side chain having a haloacetyl group, Xₐₐ₁ and Xₐₐ₃ are linked to each other, and Xₐₐ₂ is a lysine residue, an arginine residue, a cysteine residue, an aspartic acid residue, a glutamic acid residue, a 2-aminosuberic acid residue, or a diaminopropionic acid residue, and is modified with the crosslinking agent.

### Examples

Hereinafter, the present invention will be described in more detail with reference to Examples. However, the scope of the present invention is not limited to Examples.

### [Example 1] Production of ⁸⁹Zr-Pertuzumab Conjugate

### (1) Synthesis of Peptide-Conjugated Pertuzumab

A peptide having Formula (4) described below and including 17 amino acid residues was obtained by the method described in WO 2017/217347 A. The amino acid sequence of this peptide is the same as the sequence of SEQ ID NO. 2 described in paragraph [0038] of WO 2017/217347 A in a case where Xₐₐ₁ is a lysine residue, and the side chain terminal amino group of the lysine residue is conjugated with a structure of R₁ in Formula (4). A disulfide bond is formed between two cysteine residues, and an azido group is bound to the N-terminal of the peptide via a linker structure having diglycolic acid and eight PEG groups.

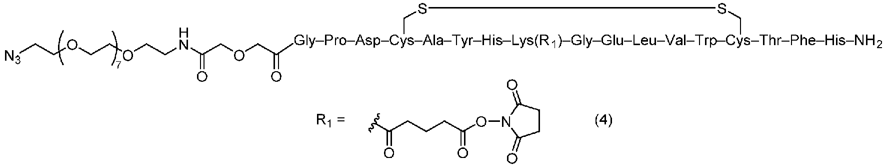

In Formula (4), Gly represents glycine, Pro represents proline, Asp represents aspartic acid, Cys represents cysteine, Ala represents alanine, Tyr represents tyrosine, His represents histidine, Lys represents lysine, Glu represents glutamic acid, Leu represents leucine, Val represents valine, Trp represents tryptophan, Phe represents phenylalanine, and Thr represents threonine.

Next, 1 mL of a pertuzumab solution (containing 30 mg of pertuzumab (trade name: Perjeta, produced by Roche)) was added into a centrifuge tube into which 6.5 mL of a 20 mmol/L acetic acid-sodium acetate buffer solution (pH: 6.0) was added. Then, 140 µL of a dimethyl sulfoxide (DMSO) solution of the peptide including 17 amino acid residues (peptide concentration: 2.4 mmol/L) was added, then pipetting was immediately performed, and the mixture was allowed to stand at room temperature and reacted for 60 minutes to obtain a solution containing peptide-conjugated pertuzumab.

Next, the solution containing peptide-conjugated pertuzumab obtained as described above was diluted with 22.5 mL of a 20 mmol/L acetic acid-sodium acetate buffer solution (pH: 6.0), the diluted solution was passed through an IgG-BP column (a column obtained by immobilizing 5632 nmol of a peptide having a sequence represented by GSGGSGPDCAYHRGELVWCTFH on a HiTrap NHS-activated HP column (manufactured by Cytiva) having a volume of 5 mL, in which peptide cysteine residues are crosslinked by a disulfide bond), and thus the antibody was retained. A 0.10 mol/L acetic acid-sodium acetate buffer solution (pH: 5.7) containing 0.15 mol/L sodium chloride was passed through the column to remove the peptide-conjugated antibody in which one molecule of pertuzumab is conjugated with two molecules of the peptide (hereinafter, referred to as "divalent peptide-conjugated pertuzumab"), and then a 0.1 mol/L glycine-hydrochloric acid buffer solution (pH: 3.5) was passed through the column to collect a fraction containing peptide-conjugated pertuzumab in which one molecule of pertuzumab is conjugated with one molecule of the peptide (hereinafter, referred to as "monovalent peptide-conjugated pertuzumab").

The fraction containing the monovalent peptide-conjugated pertuzumab was poured into an ultrafiltration filter (Amicon Ultra-15, manufactured by Merck KGaA) into which 10 mL of a pertuzumab formulation buffer solution (aqueous solution containing sucrose at a concentration of 41 g/L, L-histidine at a concentration of 3.1 g/L, and glacial acetic acid at a concentration of 657 mg/L) was added in advance, and the solution was centrifuged with a centrifuge (centrifugation conditions: 25°C, 3000 x g, 20 min). After the centrifugation, the filtrate was discarded, a pertuzumab formulation buffer solution was added again so that the total amount of the solution was 15 mL, and the second centrifugation was performed under the same conditions.

Next, a pertuzumab formulation buffer solution was added to the solution obtained as described above to obtain a peptide-conjugated pertuzumab solution in which the concentration of the monovalent peptide-conjugated pertuzumab was adjusted to 15 mg/mL (hereinafter, sometimes referred to as "solution A"). At this time, the concentration of the monovalent peptide-conjugated pertuzumab in the solution A was measured using a UV-Vis spectrophotometer (NanoDrop-2000, manufactured by Thermo Scientific Inc.) under the measurement conditions of a measurement wavelength of 280 nm, a measurement optical path length of 1 mm, and an absorbance coefficient 0.1% (280 nm) of 1.37 (an absorbance coefficient of a 1 mg/mL IgG solution at 280 nm).

### (2) Synthesis of Ligand-Conjugated Pertuzumab

DOTAGA-DBCO shown in Formula (L1-4) below was produced on the basis of the method described in Bernhard et al. DOTAGA-Anhydride: A Valuable Building Block for the Preparation of DOTA-Like Chelating Agents Chem. Eur. J. 2012, 18, 7834-7841.

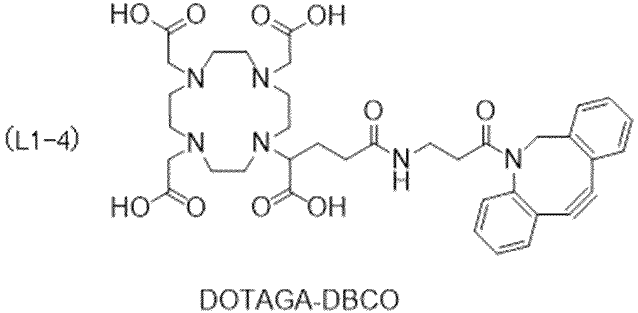

Water was added to the DOTAGA-DBCO to prepare a DOTAGA-DBCO-containing solution having a concentration of 4 mg/mL (hereinafter, sometimes referred to as "solution B").

Next, 0.6 mL of the solution A (containing 9 mg and 0.06 µmol of the monovalent peptide-conjugated pertuzumab) was added into a microtube. Then, 0.33 mL of the solution B was added and pipetting was immediately performed. The microtube was allowed to stand on a block heater set at 37°C for 1 hour, and the monovalent peptide-conjugated pertuzumab synthesized in (1) above and the DOTAGA-DBCO were subjected to a click reaction.

Next, the reaction mixture liquid obtained as described above was diluted with a pertuzumab formulation buffer solution so that the volume of the diluted liquid was 10 mL, the diluted liquid was passed through a protein A column (1 mL MabSelect SuRe, manufactured by Cytiva), and thus the peptide-conjugated antibody was retained. A 0.02 mol/L histidine buffer solution (pH: 6.0) containing 0.15 mol/L sodium chloride was passed through the column, unreacted DOTAGA-DBCO was washed, and then a 0.1 mol/L glycine-hydrochloric acid buffer solution (pH: 3.5) was passed through the column to collect a fraction containing the ligand-conjugated pertuzumab.

The total amount of the collected solution obtained above was added into an ultrafiltration filter (Amicon Ultra-15, manufactured by Merck KGaA) into which 10 mL of water for injection was added in advance, and the solution was centrifuged with a centrifuge (centrifugation conditions: 25°C, 3000 x g, 20 min). After the centrifugation, the filtrate was discarded, 13.5 mL of water for injection was added again, and centrifugation was performed under the same conditions. This centrifugation was performed twice in total, and finally, the centrifuged residue was diluted with water for injection to obtain a ligand-conjugated pertuzumab solution in which the concentration of the monovalent ligand-conjugated pertuzumab obtained by the reaction between the monovalent peptide-conjugated antibody and DOTAGA-DBCO was 15 mg/mL (hereinafter, sometimes referred to as "solution C").

### (3) Labeling Reaction

⁸⁹Zr obtained by a nuclear reaction of ⁸⁹Y(p, n)⁸⁹Zr and prepared into a ⁸⁹Zr⁴⁺ ion aqueous solution (manufactured by Nihon Medi-Physics Co., Ltd.) was added into a vial, and the solvent was distilled off.

Into the vial, 0.1 mol/L hydrochloric acid was added, pipetting was performed to wash the vial well mainly at the bottom surface, and the liquid was dispensed into a microtube so that the radioactivity was 19.8 MBq. Into the microtube, 0.1 mol/L hydrochloric acid and water for injection were added, and then a 1 mol/L sodium hydroxide aqueous solution was added to obtain 545.6 µL of a ⁸⁹Zr⁴⁺ ion solution having a pH of 3.0 (radioactivity concentration in the reaction solution: 36.3 MBq/mL). The pH was measured with a pH test paper.

To the ⁸⁹Zr⁴⁺ ion solution, 60 µL of the solution C (containing 6 nmol of the monovalent ligand-conjugated pertuzumab) was added to set the concentration of the monovalent ligand-conjugated pertuzumab in the reaction solution to 10 µmol/L, and the reaction solution was stirred with a vortex mixer. Next, the reaction solution was heated at 70°C for 30 minutes using a block heater to coordinate ⁸⁹Zr⁴⁺ ions to the ligand of the ligand-conjugated pertuzumab, and thus a ⁸⁹Zr-pertuzumab conjugate was synthesized.

The reaction mixture was extracted in an amount of 0.5 µL and separated by thin layer chromatography (iTLC-SG, manufactured by Agilent Technologies, Inc., developing solvent: mixed liquid of EDTA aqueous solution (0.1 mol/L) and hydrochloric acid (1 mol/L) (volume ratio 1 : 1)). The developed thin layer chromatogram was introduced into a TLC analyzer (GITA Star, manufactured by M&S Instruments Inc.), and the radioactivity count of the total ⁸⁹Zr including unreacted ⁸⁹Zr and the radioactivity count of the ⁸⁹Zr-pertuzumab conjugate in the reaction mixture were measured. The percentage of the radioactivity count of the ⁸⁹Zr-pertuzumab conjugate to the total ⁸⁹Zr radioactivity count was 99.3%.

### [Example 2] Production of ⁸⁹Zr-Trastuzumab Conjugate

### (1) Synthesis of Peptide-Conjugated Trastuzumab

In a centrifuge tube into which 25 mL of a 20 mmol/L acetic acid-sodium acetate buffer solution (pH: 6.0) was added, 50 mg of trastuzumab (trade name: Herceptin, produced by Roche) weighed out was dissolved to obtain a 2 mg/mL solution. Then, 233 µL of a DMSO solution of the peptide including 17 amino acid residues (concentration: 2.4 mmol/L), which was the same kind as in Example 1 (1), was added, then pipetting was immediately performed, and the mixture was allowed to stand at room temperature and reacted for 60 minutes to obtain a solution containing peptide-conjugated trastuzumab.

Next, the solution containing the peptide-conjugated trastuzumab obtained as described above was diluted with 25 mL of a 20 mmol/L acetic acid-sodium acetate buffer solution (pH: 6.0) and added to an IgG-BP column (the same as used in Example 1 (1)). A 0.10 mol/L acetic acid-sodium acetate buffer solution (pH: 5.7) containing 0.15 mol/L sodium chloride was passed through the column to remove the peptide-conjugated antibody in which one molecule of trastuzumab is conjugated with two molecules of the peptide (hereinafter, referred to as "divalent peptide-conjugated trastuzumab"), and then a 0.10 mol/L acetic acid-sodium acetate buffer solution (pH: 3.5) containing 0.15 mol/L sodium chloride was passed through the column to collect a fraction containing the peptide-conjugated antibody in which one molecule of trastuzumab is conjugated with one molecule of the peptide (hereinafter, referred to as "monovalent peptide-conjugated trastuzumab").

The fraction containing the monovalent peptide-conjugated trastuzumab was poured into an ultrafiltration filter (Amicon Ultra-15, manufactured by Merck KGaA) into which a trastuzumab formulation buffer solution (19 g/L trehalose hydrate, 470 mg/L L-histidine hydrochloride, 300 mg/L L-histidine) was added in advance, and the solution was centrifuged with a centrifuge (centrifugation conditions: 20°C, 2330 x g, 20 min). After the centrifugation, the filtrate was discarded, a trastuzumab formulation buffer solution was added again so that the total amount of the solution was 15 mL, and the second centrifugation was performed under the same conditions. The addition of a trastuzumab formulation buffer solution, the extraction of the filtrate, and the centrifugation were performed three times in total.

Next, a trastuzumab formulation buffer solution was added to the solution obtained as described above to obtain a peptide-conjugated trastuzumab solution in which the concentration of the monovalent peptide-conjugated trastuzumab was adjusted to 15 mg/mL (hereinafter, sometimes referred to as "solution D"). At this time, the concentration of the monovalent peptide-conjugated trastuzumab in the solution D was measured as described above using a UV-Vis spectrophotometer (NanoDrop-2000, manufactured by Thermo Scientific Inc.).

### (2) Synthesis of Ligand-Conjugated Trastuzumab

Into a microtube, 0.6 mL of the solution D (containing 9 mg and 0.06 µmol of the monovalent peptide-conjugated trastuzumab) was added. Then, 0.33 mL of the solution B prepared in the same manner as in Example 1 (2) was added, and pipetting was immediately performed. The microtube was allowed to stand on a block heater set at 37°C for 1 hour, and the peptide-conjugated trastuzumab synthesized in (1) above and DOTAGA-DBCO were subjected to a click reaction.

Next, the reaction mixture liquid obtained as described above was diluted with a trastuzumab formulation buffer solution so that the volume of the diluted liquid was 10 mL, the diluted liquid was passed through a protein A column (1 mL MabSelect SuRe, manufactured by Cytiva), and thus the antibody was retained. A 0.02 mol/L histidine buffer solution (pH: 6.0) containing 0.15 mol/L sodium chloride was passed through the column, unreacted DOTAGA-DBCO was washed, and then a 0.1 mol/L glycine-hydrochloric acid buffer solution (pH: 3.5) was passed through the column to collect a fraction containing the ligand-conjugated trastuzumab.

The total amount of the collected solution obtained above was added into an ultrafiltration filter (Amicon Ultra-15, manufactured by Merck KGaA) into which 10 mL of water for injection was added in advance, and the solution was centrifuged with a centrifuge (centrifugation conditions: 25°C, 3000 x g, 20 min). After the centrifugation, the filtrate was discarded, 13.5 mL of water for injection was added again, and centrifugation was performed under the same conditions. This centrifugation was performed 4 times in total, and finally, the centrifuged residue was diluted with water for injection to obtain a ligand-conjugated trastuzumab solution in which the concentration of the monovalent ligand-conjugated trastuzumab obtained by the reaction between the monovalent peptide-conjugated antibody and DOTAGA-DBCO was 15 mg/mL (hereinafter, sometimes referred to as "solution E").

### (3) Labeling Reaction

⁸⁹Zr⁴⁺ ions were coordinated to the ligand of the ligand-conjugated trastuzumab to synthesize a ⁸⁹Zr-trastuzumab conjugate in the same manner as in Example 1 (3) except that 545.6 µL of a ⁸⁹Zr⁴⁺ ion solution containing ⁸⁹Zr⁴⁺ ions at 36.3 MBq/mL and having a pH of 3.0 was used and the solution C was changed to the solution E. As a result of analyzing the reaction mixture by a thin layer chromatography method in the same manner as in Example 1 (3), the percentage of the radioactivity count of the ⁸⁹Zr-trastuzumab conjugate to the total ⁸⁹Zr radioactivity count was 99.5%.

### [Example 3] Production of ²²⁵Ac-Pertuzumab Conjugate

### (1) Synthesis of Ligand-Conjugated Pertuzumab

In accordance with the same procedure as in Examples 1 (1) and 1 (2), ligand-conjugated pertuzumab was synthesized to obtain a solution C.

### (2) Labeling Reaction

Into a vial containing ²²⁵Ac(NO₃)₃ purchased from Oak Ridge National Laboratory, 0.2 mol/L hydrochloric acid was added, pipetting was performed to wash the vial well mainly at the bottom surface, and the liquid was dispensed into a microtube so that the radioactivity was 0.332 MBq. Into the microtube, 0.2 mol/L hydrochloric acid and water for injection were added, and then a 1 mol/L sodium hydroxide aqueous solution was added to obtain 276 µL of an ²²⁵Ac³⁺ ion solution having a pH of 7.0 (radioactivity concentration in the reactionsolution: 1.2 MBq/mL). The pH was measured with a pH test paper.

To the ²²⁵Ac³⁺ ion solution, 30 µL of the solution C (containing 3 nmol of the monovalent ligand-conjugated pertuzumab) was added to set the concentration of the monovalent ligand-conjugated pertuzumab in the reaction solutionto 9.8 µmol/L, and the reaction liquid was stirred with a vortex mixer. Next, the reaction liquid was heated at 40°C for 30 minutes using a block heater to coordinate ²²⁵Ac³⁺ ions to the ligand of the ligand-conjugated pertuzumab, and thus an ²²⁵Ac-pertuzumab conjugate was synthesized.

The reaction mixture was extracted in an amount of 2 µL and separated by thin layer chromatography (iTLC-SG, manufactured by Agilent Technologies, Inc., developing solvent: mixed liquid of EDTA aqueous solution (0.1 mol/L) and hydrochloric acid (1 mol/L) (volume ratio 1 : 1)). The developed thin layer chromatogram was introduced into a TLC analyzer (GITA Star, manufactured by M&S Instruments Inc.), and the radioactivity count of the total ²²⁵Ac including unreacted ²²⁵Ac and the radioactivity count of the ²²⁵Ac-pertuzumab conjugate in the reaction mixture were measured. The percentage of the radioactivity count of the ²²⁵Ac-pertuzumab conjugate to the total ²²⁵Ac radioactivity count was 91.0%.

### [Example 4] Production of ²²⁵Ac-Trastuzumab Conjugate

### (1) Synthesis of Ligand-Conjugated Trastuzumab

In accordance with the same procedure as in Examples 2 (1) and 2 (2), ligand-conjugated trastuzumab was synthesized to obtain a solution E.

### (2) Labeling Reaction

²²⁵Ac ions were coordinated to the ligand of the ligand-conjugated trastuzumab to synthesize an ²²⁵Ac-trastuzumab conjugate in the same manner as in Example 3 (2) except that 276 µL of an ²²⁵Ac³⁺ ion solution having a pH of 7.0 (radioactivity concentration in the reaction solution: 0.98 MBq/mL) was used and the solution C was changed to the solution E. As a result of analyzing the reaction mixture by a thin layer chromatography method in the same manner as in Example 3 (2), the percentage of the radioactivity count of the ²²⁵Ac-trastuzumab conjugate to the total ²²⁵Ac radioactivity count was 83.6%.

### [Example 5] Production of ²²⁵Ac-Pertuzumab Conjugate under Stabilizer-Added Condition

### (1) Synthesis of Ligand-ConjugatedPertuzumab

In accordance with the same procedure as in Examples 1 (1) and 1 (2), ligand-conjugated pertuzumab was synthesized to obtain a solution C.

### (2) Labeling Reaction

²²⁵Ac ions were coordinated to the ligand of the ligand-conjugated pertuzumab to synthesize an ²²⁵Ac-pertuzumab conjugate in the same manner as in Example 3 (2) except that 186 µL of an ²²⁵Ac³⁺ ion solution having a pH of 7.0 (radioactivity concentration in the reaction solution: 1.5 MBq/mL) was used and 90 µL of a 500 mmol/L sucrose aqueous solution was added to the reaction solution.

As a result of analyzing the reaction mixture by a thin layer chromatography method in the same manner as in Example 3 (2), the percentage of the radioactivity count of the ²²⁵Ac-pertuzumab conjugate to the total ²²⁵Ac radioactivity count was 91.0%.

### [Example 6] Production of ²²⁵Ac-Trastuzumab Conjugate under Acidic Condition

### (1) Synthesis of Ligand-Conjugated Trastuzumab

In accordance with the same procedure as in Examples 2 (1) and 2 (2), ligand-conjugated trastuzumab was synthesized to obtain a solution (E).

### (2) Labeling Reaction

Into a vial containing ²²⁵Ac(NO₃)₃ purchased from Oak Ridge National Laboratory, 0.2 mol/L hydrochloric acid was added, pipetting was performed to wash the vial well mainly at the bottom surface, and the liquid was dispensed into a microtube so that the radioactivity was 0.301 MBq. Into the microtube, 0.2 mol/L hydrochloric acid and water for injection were added, and then a 1 mol/L sodium hydroxide aqueous solution was added to obtain 272 µL of an ²²⁵Ac³⁺ ion solution having a pH of 4.0 (radioactivity concentration in the reaction solution: 1.1 MBq/mL). The pH was measured with a pH test paper.

An ²²⁵Ac-trastuzumab conjugate was synthesized in the same manner as in Example 4 (2) except that the above-described ²²⁵Ac³⁺ ion solution was used as an ²²⁵Ac³⁺ ion solution. As a result of analyzing the reaction mixture by a thin layer chromatography method, the percentage of the radioactivity count of the ²²⁵Ac-trastuzumab conjugate to the total ²²⁵Ac radioactivity count was 93.0%.

### [Comparative Example 1] Production of ⁸⁹Zr-Pertuzumab Conjugate with Two-Step Method

### (1) Production of ⁸⁹Zr-labeled DOTAGA-DBCO

To 20 µL of a 0.1 mol/L hydrochloric acid containing ⁸⁹Zr⁴⁺ ions having a radioactivity of 19.9 MBq, 20 µL of an aqueous solution (containing 6 nmol of a ligand) containing DOTAGA-DBCO (concentration: 0.3 mmol/L) and sodium acetate (concentration: 0.156 mol/L) and 20 µL of an aqueous solution containing gentisic acid (concentration: 0.15 mol/L) and sodium acetate (concentration: 0.156 mol/L) were added, and the mixture was reacted at 70°C for 60 minutes to coordinate DOTAGA-DBCO to ⁸⁹Zr⁴⁺. The obtained reaction mixture of ⁸⁹Zr-labeled DOTAGA-DBCO was extracted in an amount of 0.5 µL and separated by thin layer chromatography (iTLC-SG, manufactured by Agilent Technologies, Inc., developing solvent: mixed liquid of water for injection and acetonitrile (volume ratio 1 : 1)). The developed thin layer chromatogram was introduced into a TLC analyzer (GITA Star, manufactured by M&S Instruments Inc.), and the radioactivity count of the total ⁸⁹Zr including unreacted ⁸⁹Zr and the radioactivity count of ⁸⁹Zr-labeled DOTAGA-DBCO in the reaction mixture were measured. The percentage of the radioactivity count of ⁸⁹Zr-labeled DOTAGA-DBCO to the total ⁸⁹Zr radioactivity count was 98.7%.

### (2) Peptide-Conjugated Pertuzumab

In accordance with the same procedure as in Example 1 (1), peptide-conjugated pertuzumab was synthesized.

### (3) Production of ⁸⁹Zr-Pertuzumab Conjugate

To 90 µL of the ⁸⁹Zr-labeled DOTAGA-DBCO solution obtained above, 0.06 mL of the solution A (containing 6 nmol of the monovalent antibody) was added and reacted at 37°C for 90 minutes, and the antibody and ⁸⁹Zr-labeled DOTAGA-DBCO were bound by a click reaction to obtain a ⁸⁹Zr-pertuzumab conjugate by a production method including two steps of a step of labeling a ligand with ⁸⁹Zr and a step of a click reaction of a ⁸⁹Zr ligand. As a result of measuring the radioactivity count of the total ⁸⁹Zr including unreacted ⁸⁹Zr and the radioactivity count of the ⁸⁹Zr-pertuzumab conjugate in the obtained reaction mixture of the ⁸⁹Zr-pertuzumab conjugate in the same manner as in Example 1 (3), the percentage of the radioactivity count of the ⁸⁹Zr-pertuzumab conjugate to the total ⁸⁹Zr radioactivity count was 82.4%.

### [Comparative Example 2] Production of ⁸⁹Zr-Trastuzumab Conjugate with Two-Step Method

### (1) Production of ⁸⁹Zr-labeled DOTAGA-DBCO

In accordance with the same procedure as in Comparative Example 1 (1), ⁸⁹Zr-labeled DOTAGA-DBCO was produced using ⁸⁹Zr⁴⁺ ions having a radioactivity of 19.8 MBq. The obtained reaction mixture of ⁸⁹Zr-labeled DOTAGA-DBCO was extracted in an amount of 0.5 µL and separated by thin layer chromatography (iTLC-SG, manufactured by Agilent Technologies, Inc., developing solvent: mixed liquid of water for injection and acetonitrile (volume ratio 1 : 1)). The developed thin layer chromatogram was introduced into a TLC analyzer (GITA Star, manufactured by M&S Instruments Inc.), and the radioactivity count of the total ⁸⁹Zr including unreacted ⁸⁹Zr and the radioactivity count of ⁸⁹Zr-labeled DOTAGA-DBCO in the reaction mixture were measured. The percentage of the radioactivity count of ⁸⁹Zr-labeled DOTAGA-DBCO to the total ⁸⁹Zr radioactivity count was 98.6%.

### (2) Peptide-Conjugated Trastuzumab

In accordance with the same procedure as in Example 2 (1), peptide-conjugated trastuzumab was synthesized.

### (3) Production of ⁸⁹Zr-Trastuzumab Conjugate

To 90 µL of the ⁸⁹Zr-labeled DOTAGA-DBCO solution obtained above, 0.06 mL of the solution D (containing 6 nmol of the monovalent antibody) was added and reacted at 37°C for 90 minutes, and the antibody and ⁸⁹Zr-labeled DOTAGA-DBCO were bound to obtain a ⁸⁹Zr-trastuzumab conjugate. As a result of measuring the radioactivity count of the total ⁸⁹Zr including unreacted ⁸⁹Zr and the radioactivity count of the ⁸⁹Zr-trastuzumab conjugate in the obtained reaction mixture of the ⁸⁹Zr-trastuzumab conjugate in the same manner as in Example 1 (3), the percentage of the radioactivity count of the ⁸⁹Zr-trastuzumab conjugate to the total ⁸⁹Zr radioactivity count was 81.7%.

### [Comparative Example 3] Production of ²²⁵Ac-Pertuzumab Conjugate with Two-Step Method

### (1) Production of ²²⁵Ac-labeled DOTAGA-DBCO

To 20 µL of a 0.2 mol/L hydrochloric acid containing ²²⁵Ac³⁺ ions having a radioactivity of 0.6 MBq, 40 µL of an aqueous solution containing DOTAGA-DBCO (concentration: 0.3 mmol/L) and sodium acetate (concentration: 0.156 mol/L) and 20 µL of sodium acetate aqueous solution (concentration: 0.156 mol/L) were added, and the mixture was reacted at 70°C for 60 minutes to coordinate ²²⁵Ac³⁺ to DOTAGA-DBCO. The obtained reaction mixture of ²²⁵Ac-labeled DOTAGA-DBCO was extracted in an amount of 2 µL and separated by thin layer chromatography (iTLC-SG, manufactured by Agilent Technologies, Inc., developing solvent: mixed liquid of water for injection and acetonitrile (volume ratio 1 : 1)). The developed thin layer chromatogram was introduced into a TLC analyzer (GITA Star, manufactured by M&S Instruments Inc.), and the radioactivity count of the total ²²⁵Ac including unreacted ²²⁵Ac and the radioactivity count of ²²⁵Ac-labeled DOTAGA-DBCO in the reaction mixture were measured. The percentage of the radioactivity count of ²²⁵Ac-labeled DOTAGA-DBCO to the total ²²⁵Ac radioactivity count was 98.9%.

### (2) Peptide-Conjugated Pertuzumab

In accordance with the same procedure as in Example 1 (1), peptide-conjugated pertuzumab was synthesized.

### (3) Production of ²²⁵Ac-Pertuzumab Conjugate

To 40 µL of the ²²⁵Ac-labeled DOTAGA-DBCO solution obtained above, 0.06 mL of the solution A (containing 6 nmol of the monovalent antibody) was added and reacted at 37°C for 90 minutes, and the antibody and ²²⁵Ac-labeled DOTAGA-DBCO were bound to obtain an ²²⁵Ac-pertuzumab conjugate. The obtained reaction mixture of the ²²⁵Ac-pertuzumab conjugate was extracted in an amount of 2 µL and separated by thin layer chromatography (iTLC-SG, manufactured by Agilent Technologies, Inc., developing solvent: mixed liquid of EDTA aqueous solution (0.1 mol/L) and acetonitrile (volume ratio 1 : 1)). The developed thin layer chromatogram was introduced into a TLC analyzer (GITA Star, manufactured by M&S Instruments Inc.), and the radioactivity count of the total ²²⁵Ac including unreacted ²²⁵Ac and the radioactivity count of the ²²⁵Ac-pertuzumab conjugate in the reaction mixture were measured. The percentage of the radioactivity count of the ²²⁵Ac-pertuzumab conjugate to the total ²²⁵Ac radioactivity count was 67.3%.

### [Comparative Example 4] Production of ²²⁵Ac-Trastuzumab Conjugate with Two-Step Method

### (1) Production of ²²⁵Ac-labeled DOTAGA-DBCO

In accordance with the same procedure as in Comparative Example 3 (1), ²²⁵Ac-labeled DOTAGA-DBCO was produced. The obtained reaction mixture of ²²⁵Ac-labeled DOTAGA-DBCO was extracted in an amount of 2 µL and separated by thin layer chromatography (iTLC-SG, manufactured by Agilent Technologies, Inc., developing solvent: mixed liquid of water for injection and acetonitrile (volume ratio 1 : 1)). The developed thin layer chromatogram was introduced into a TLC analyzer (GITA Star, manufactured by M&S Instruments Inc.), and the radioactivity count of the total ²²⁵Ac including unreacted ²²⁵Ac and the radioactivity count of ²²⁵Ac-labeled DOTAGA-DBCO in the reaction mixture were measured. The percentage of the radioactivity count of ²²⁵Ac-labeled DOTAGA-DBCO to the total ²²⁵Ac radioactivity count was 98.8%.

### (2) Peptide-Conjugated Trastuzumab

In accordance with the same procedure as in Example 2 (1), peptide-conjugated trastuzumab was synthesized.

### (3) Production of ²²⁵Ac-Trastuzumab Conjugate

To 40 µL of the ²²⁵Ac-labeled DOTAGA-DBCO solution obtained above, 0.06 mL of the solution D (containing 6 nmol of the monovalent antibody) was added and reacted at 37°C for 90 minutes, and the antibody and ²²⁵Ac-labeled DOTAGA-DBCO were bound to obtain an ²²⁵Ac-trastuzumab conjugate. The obtained reaction mixture of the ²²⁵Ac-trastuzumab conjugate was extracted in an amount of 2 µL and separated by thin layer chromatography (iTLC-SG, manufactured by Agilent Technologies, Inc., developing solvent: mixed liquid of EDTA aqueous solution (0.1 mol/L) and acetonitrile (volume ratio 1 : 1)). The developed thin layer chromatogram was introduced into a TLC analyzer (GITA Star, manufactured by M&S Instruments Inc.), and the radioactivity count of the total ²²⁵Ac including unreacted ²²⁵Ac and the radioactivity count of the ²²⁵Ac-trastuzumab conjugate in the reaction mixture were measured. The percentage of the radioactivity count of the ²²⁵Ac-trastuzumab conjugate to the total ²²⁵Ac radioactivity count was 67.2%.

In all of Examples 1 to 6 in which antibody labeling was performed in one step, a high labeling rate was exhibited. Meanwhile, in Comparative Examples 1 to 4 in which labeling was performed in two steps, the labeling rate was low. Thus, it has been shown that the production method with one step is superior to the production method with two steps.

### [Comparative Example 5] Production of ²²⁵Ac-Trastuzumab Conjugate with One-Step Method (Condition Setting with Reference to US 2015/0157742 A)

### (1) Ligand-Conjugated Trastuzumab

In accordance with the same procedure as in Examples 2 (1) and 2 (2), ligand-conjugated trastuzumab was synthesized. The synthesized ligand-conjugated trastuzumab was added into an ultrafiltration filter (Amicon Ultra-15, manufactured by Merck KGaA) into which 10 mL of a 0.15 mol/L sodium chloride-50 mmol/L sodium acetate buffer solution (pH: 7.2) was added in advance, and the solution was centrifuged with a centrifuge (centrifugation conditions: 25°C, 3000 x g, 20 min). After the centrifugation, the filtrate was discarded, 13.5 mL of a 0.15 mol/L sodium chloride-50 mmol/L sodium acetate buffer solution (pH: 7.2) was added again, and centrifugation was performed under the same conditions. This centrifugation was performed 4 times in total, and finally, the centrifuged residue was diluted with a 0.15 mol/L sodium chloride-50 mmol/L sodium acetate buffer solution (pH: 7.2) to obtain a ligand-conjugated antibody solution having a monovalent antibody concentration of 5 mg/mL (hereinafter, sometimes referred to as "solution F").

### (2) Production of ²²⁵Ac-Trastuzumab Conjugate

With Reference to the conditions disclosed in the prior art documents, an ²²⁵Ac-trastuzumab conjugate was produced using the ligand-conjugated trastuzumab of (1) as follows.

Into a vial containing ²²⁵Ac(NO₃)₃ purchased from Oak Ridge National Laboratory, 0.2 mol/L hydrochloric acid was added, pipetting was performed to wash the vial well mainly at the bottom surface, and the liquid was dispensed into a microtube so that the radioactivity was 0.288 MBq (the liquid amount was 5 µL). Into the microtube, 5 µL of water for injection and 500 µL of a 100 mmol/L Tris buffer solution (pH: 9.0) were added to obtain 510 µL of an ²²⁵Ac³⁺ ion solution having a pH of 9.0 (radioactivity concentration in the reaction solution: 0.56 MBq/mL). The pH was measured with a pH test paper.

To the ²²⁵Ac³⁺ ion solution, 20 µL of the solution F was added, the mixture was heated at 40°C for 15 minutes to synthesize an ²²⁵Ac-trastuzumab conjugate, and the reaction mixture was analyzed by a thin layer chromatography method in the same manner as in Example 3 (2). Thus, the percentage of the radioactivity count of the ²²⁵Ac-trastuzumab conjugate to the total ²²⁵Ac radioactivity count was measured. The result was 10.0%.

### [Comparative Example 6] Production of ²²⁵Ac-Trastuzumab Conjugate with One-Step Method (US 2012/0220754 A)

### (1) Ligand-Conjugated Trastuzumab

In accordance with the same procedure as in Examples 2 (1) and 2 (2), ligand-conjugated trastuzumab was synthesized. The synthesized ligand-conjugated trastuzumab was added into an ultrafiltration filter (Amicon Ultra-15, manufactured by Merck KGaA) into which 10 mL of physiological saline was added in advance, and the solution was centrifuged with a centrifuge (centrifugation conditions: 25°C, 3000 x g, 20 min). After the centrifugation, the filtrate was discarded, 13.5 mL of physiological saline was added again, and centrifugation was performed under the same conditions. This centrifugation was performed 4 times in total, and finally, the centrifuged residue was diluted with physiological saline to obtain a ligand-conjugated antibody solution having a monovalent antibody concentration of 10 mg/mL (hereinafter, sometimes referred to as "solution G").

### (2) Production of ²²⁵Ac-Trastuzumab Conjugate

With Reference to the conditions disclosed in the prior art documents, an ²²⁵Ac-trastuzumab conjugate was produced using the ligand-conjugated trastuzumab of (1) as follows.

Into a vial containing ²²⁵Ac(NO₃)₃ purchased from Oak Ridge National Laboratory, 0.2 mol/L hydrochloric acid was added, pipetting was performed to wash the vial well mainly at the bottom surface, and the liquid was dispensed into a microtube so that the radioactivity was 0.272 MBq (the liquid amount was 75 µL). Into the microtube, 225 µL of water for injection, 100 µL of a 3 mol/L sodium acetate buffer solution, and 20 µL of a saturated gentisic acid solution were added to obtain 420 µL of an ²²⁵Ac³⁺ ion solution (radioactivity concentration in the reaction solution: 0.65 MBq/mL). The pH was measured with a pH test paper and confirmed to be in the range of 5.5 to 7.0.

To the ²²⁵Ac³⁺ ion solution, 100 µL of the solution G was added, the mixture was heated at 37°C for 90 minutes to synthesize an ²²⁵Ac-trastuzumab conjugate, and the reaction mixture was analyzed by a thin layer chromatography method in the same manner as in Example 3 (2). Thus, the percentage of the radioactivity count of the ²²⁵Ac-trastuzumab conjugate to the total ²²⁵Ac radioactivity count was measured. The result was 17.9%.

In all of Examples 3 to 6 in which ²²⁵Ac labeling was performed in one step, a high labeling rate was exhibited. Meanwhile, in Comparative Examples 5 to 6 in which labeling was performed under the conditions set on the basis of the conditions in the prior art documents, the labeling rate was low. Thus, it has been shown that the production method with one step of the present invention is superior to the production method according to the prior art documents.

### [Example 7] Production of ⁸⁹Zr-Trastuzumab Conjugate under Aggregation Inhibitor-Added Condition

### (1) Synthesis of Ligand-Conjugated Trastuzumab

In accordance with the same procedure as in Examples 2 (1) and 2 (2), ligand-conjugated trastuzumab was synthesized to obtain a solution E.

### (2) Labeling Reaction

⁸⁹Zr obtained by a nuclear reaction of ⁸⁹Y(p, n)⁸⁹Zr and prepared into a ⁸⁹Zr⁴⁺ ion aqueous solution (manufactured by Nihon Medi-Physics Co., Ltd.) was added into a vial, and the solvent was distilled off.

Into the vial, 814 µL of 0.1 mol/L hydrochloric acid was added, pipetting was performed to wash the vial well mainly at the bottom surface, and a solution containing ⁸⁹Zr⁴⁺ ions at 1000 MBq/mL was obtained. Into a microtube, 20 µL of this solution was dispensed, 40 µL of 0.1 mol/L hydrochloric acid, 300 µL of an aggregation inhibitor that is water for injection or an aqueous solution of sorbitol, xylose, glucose, trehalose, or sucrose adjusted to 300 mmoL/L, and 210 µL of water for injection were added, and then a 1 mol/L sodium hydroxide aqueous solution was added to obtain 570 µL of a ⁸⁹Zr⁴⁺ ion solution having a pH of 3.0 (radioactivity concentration in the reactionsolution: 35 MBq/mL). The pH was measured with a pH test paper.

To the ⁸⁹Zr⁴⁺ ion solution, 30 µL of the solution E (containing 3 nmol of the monovalent ligand-conjugated trastuzumab) was added to set the concentration of the monovalent ligand-conjugated trastuzumab in the reaction solution to 5 µmol/L, and the reaction solution was stirred with a vortex mixer. Next, the reaction solution was heated at 70°C for 30 minutes using a block heater to coordinate ⁸⁹Zr⁴⁺ ions to the ligand of the ligand-conjugated trastuzumab, and thus a ⁸⁹Zr-trastuzumab conjugate was synthesized.

### (Evaluation)

In size exclusion chromatography (SEC) analysis, a chromatogram was acquired using an RI detector, and the labeling rate was evaluated. Furthermore, in the SEC analysis, a chromatogram was acquired using a UV detector, and the proportion of aggregates was evaluated to verify the effect of stabilizing the antibody by the aggregation inhibitor. The analysis was performed under the following conditions using an e2695 separation module manufactured by Waters Corporation as a liquid chromatography apparatus, a 2489 UV/Vis detector manufactured by Waters Corporation as a UV detector, and a Gabi Star detector manufactured by Raytest as an RI detector. The area percentage of the peak area value of the ⁸⁹Zr trastuzumab conjugate to the total peak area value in the chromatogram obtained using an RI detector was regarded as the labeling rate. In the chromatogram obtained using a UV detector, a peak detected other than the peak of the ⁸⁹Zr trastuzumab conjugate (hereinafter, sometimes referred to as the "main peak") was regarded as a peak of an aggregate.

Table 1 shows the labeling rates of each component and Table 2 shows the proportions of each component after the end of production (0-day point) and after storage for 14 days. Because of the lower concentration of the ligand-conjugated antibody, the labeling rate was lower than in Example 2, but in all cases, the labeling rate was higher than 50%. Thus, even in the case of adding an aggregation inhibitor, the labeling rate was not lower by 20% or more than in the case of not adding an aggregation inhibitor. After the storage for 14 days, formation of aggregates was suppressed in the case of adding an aggregation inhibitor as compared with the case of not adding an aggregation inhibitor.

### [HPLC conditions]

Column: TOSOH TSKgel guardcolumn SWXL (6 mm × 4 cm), TOSOH TSKgel G3000SWXL (5 µm, 7.8 x 30 cm)
Column temperature: constant temperature around 25°C
Mobile phase: 0.2 mol/L arginine hydrochloride-containing 0.1 mol/L sodium dihydrogen phosphate buffer solution (pH: 6.8)
Flow rate: 1.0 mL per minute
Area measurement range: 15 minutes
Detection wavelength (UV): 280 nm
Detection range (RI): 50 to 803 keV

**[Table 1]**

| Aggregation inhibitor | Measurement date | Labeling rate (%) |
|---|---|---|
| - | 0-Day point | 71.5 |
| | 14-Day point | 63.6 |
| Sorbitol | 0-Day point | 52.5 |
| | 14-Day point | 57.4 |
| Xylose | 0-Day point | 73.7 |
| | 14-Day point | 71.4 |
| Glucose | 0-Day point | 54.3 |
| | 14-Day point | 65.1 |
| Trehalose | 0-Day point | 81.7 |
| | 14-Day point | 74.6 |
| Sucrose | 0-Day point | 85.3 |
| | 14-Day point | 72.7 |

**[Table 2]**

| Aggregation inhibitor | Measurement date | Proportion of main peak (%) | Proportion of peak of aggregates (%) |
|---|---|---|---|
| - | 0-Day point | 97.1 | 2.9 |
| | 14-Day point | 89.0 | 11.0 |
| Sorbitol | 0-Day point | 97.1 | 2.9 |
| | 14-Day point | 94.5 | 5.5 |
| Xylose | 0-Day point | 95.7 | 4.3 |
| | 14-Day point | 95.6 | 4.4 |
| Glucose | 0-Day point | 96.2 | 3.8 |
| | 14-Day point | 95.6 | 4.4 |
| Trehalose | 0-Day point | 96.3 | 3.7 |
| | 14-Day point | 98.3 | 1.7 |
| Sucrose | 0-Day point | 95.0 | 5.0 |
| | 14-Day point | 92.4 | 7.6 |

### [Evaluation of Antigen-Binding Activity]

The antigen-binding activity was confirmed by in vitro autoradiography (ARG). Five × 10⁶ cells of SK-OV-3 cells, which are HER2-positive human ovarian cancer cell lines, and 1 × 10' cells of MDA-MB-231 cells, which are HER2-negative human breast cancer cell lines, purchased from American Type Culture Collection (ATCC) were administered subcutaneously in the flank of female BALB/c nu/nu mice (available from Charles River Laboratories Japan, Inc.) to prepare tumor-bearing mice. Then, the SK-OV-3 tumor and the MDA-MB-231 tumor were extracted and embedded in Tissue-Tek O.C.T. Compound (manufactured by Sakura Finetek Japan Co., Ltd.) to prepare frozen sections. The radioconjugates obtained in Example 1 and Comparative Example 1 were each added to 1% bovine serum albumin-containing PBS so that the radioactivity concentration was 5 kBq/mL, and the SK-OV-3 tumor section and the MDA-MB-231 tumor section were immersed. Each section was contacted with an imaging plate and then read by a scanner type image analyzer (Typhoon FLA 7000, manufactured by GE Healthcare Japan), and the amount of radioactivity bound to the section was evaluated. Fig. 1 shows the results. The vertical axis of the graph represents a value obtained as follows. The count value in an ROI set in the used tumor section was divided by the area of the ROI, and the quotient was corrected by a value obtained by dividing the count value of a standard radiation source by the area of the ROI. The resulting value was represented by the vertical axis. The horizontal axis represents the cell type of the tumor section used for evaluation. The graph shows the average ± standard deviation in the ROIs set in each sample. In the radioconjugates produced in accordance with the descriptions of Example 1 and Comparative Example 1, the binding activity to HER2 was confirmed. The radioconjugates produced in accordance with Example 1 and Comparative Example 1 bound only to SK-OV-3 tumor sections, and the HER2-selective binding activity was confirmed.

The present application is based on Japanese Patent Application No. 2022-057723 filed in Japan (filing date: March 30, 2022), the contents of which are all incorporated herein.

## Claims

1. A method for producing a conjugate of a radioactive metal nuclide and an antibody, the method comprising:
a step of reacting a radioactive metal nuclide with an antibody conjugated with a ligand compound in a reaction solution containing water to coordinate the radioactive metal nuclide to the ligand compound, wherein
the reaction solution does not contain a pH buffer and has a pH of 2 or more and 10 or less.

2. The method according to claim 1, wherein the radioactive metal nuclide is at least one selected from ⁶⁸Ga, ⁸⁹Zr, ⁹⁰Y, ¹¹¹In, ¹⁷⁷Lu, and ²²⁵Ac.

3. The method according to claim 2, wherein the radioactive metal nuclide is ⁸⁹Zr, and the reaction solution has a pH of 2 or more and 4 or less.

4. The method according to claim 2, wherein the radioactive metal nuclide is ²²⁵Ac, and the reaction solution has a pH of 5 or more and 10 or less.

5. The method according to claim 1 or 2, wherein a concentration of the antibody in the reaction solution is 1 µmol/L or more and 2000 µmol/L or less.

6. The method according to claim 1 or 2, wherein the reaction solution is heated to 30°C or higher and 80°C or lower to coordinate the radioactive metal nuclide to the ligand compound.

7. The method according to claim 1 or 2, wherein the reaction solution contains at least one aggregation inhibitor selected from the group of saccharides, the group consisting of sucrose, trehalose, mannitol, maltose, lactose, arabinose, xylose, sorbitol, fructose, glucose, mannose, melezitose, and nystose.

8. The method according to claim 1 or 2, wherein the ligand compound has Formula (1) described below: 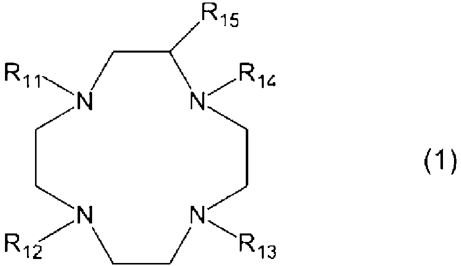 wherein R₁₁, R₁₃, and R₁₄ each independently represent a group including - (CH₂)ₚCOOH, -(CH₂)ₚC₅H₄N, -(CH₂)ₚPO₃H₂, -(CH₂)ₚCONH₂, or-(CHCOOH)(CH₂)ₚCOOH, one of R₁₂ or R₁₅ represents a hydrogen atom, a carboxyl group, or a carboxyalkyl group having 2 or 3 carbon atoms, another of R₁₂ or R₁₅ represents a substituent that is to be conjugated with the antibody, p is an integer of 0 or more and 3 or less, R₁₅ is a hydrogen atom when R₁₂ is a substituent that is to be conjugated with the antibody, and R₁₅ is a substituent that is to be conjugated with the antibody when R₁₂ is not a substituent that is to be conjugated with the antibody.

9. The method according to claim 1 or 2, wherein an Fc region of the antibody is site-specifically conjugated with the ligand compound via a linker.

10. The method according to claim 9, wherein
the antibody is a human IgG antibody, and
the linker includes a peptide including 13 or more and 17 or less amino acid residues and is formed by a crosslinking reaction between the peptide conjugated with a crosslinking agent and the antibody,
the peptide having Formula (Y) below:
(Xₐ)-Xₐₐ₁-(X_{b})-Xₐₐ₂-(X_{c})-Xₐₐ₃-(X_{d}) ·· (Y)
wherein Xₐ, X_{b}, X_{c}, and X_{d} represent consecutive Xs of which number is a, consecutive Xs of which number is b, consecutive Xs of which number is c, and consecutive Xs of which number is d, respectively, X represents an amino acid residue having a side chain having neither a thiol group nor a haloacetyl group, a, b, c, and d are each independently an integer of 1 or more and 5 or less and satisfy a + b + c + d ≤ 14, Xₐₐ₁ and Xₐₐ₃ each independently represent an amino acid residue derived from an amino acid having a side chain having a thiol group, or one of Xₐₐ₁ or Xₐₐ₃ represents an amino acid residue derived from an amino acid having a side chain having a thiol group and another of Xₐₐ₁ or Xₐₐ₃ represents an amino acid residue derived from an amino acid having a side chain having a haloacetyl group, Xₐₐ₁ and Xₐₐ₃ are linked to each other, and Xₐₐ₂ is a lysine residue, an arginine residue, a cysteine residue, an aspartic acid residue, a glutamic acid residue, a 2-aminosuberic acid residue, or a diaminopropionic acid residue, and is modified with the crosslinking agent.
